# EUROPÄISCHE PATENTSCHRIFT

(11) **EP 1 993 493 B1**
(45) Veröffentlichungstag und Bekanntmachung des Hinweises auf die Patenterteilung: **20.09.2017**
(21) Anmeldenummer: 07711804.0
(22) Anmeldetag: 06.03.2007
(51) Int. Cl.: A61F 9/01, A61G 13/00, A61F 9/00, G02B 27/00, A61B 3/00, A61B 3/18, A61B 90/00

(54) **SYSTEM ZUR BEHANDLUNG ODER DIAGNOSE AM AUGE**
SYSTEM FOR THE TREATMENT OR DIAGNOSIS OF AN EYE
SYSTÈME DE TRAITEMENT OU DE DIAGNOSTIC DE L'OEIL

(30) Priorität: 10.03.2006 DE 102006011623; 10.11.2006 DE 102006053580
(43) Veröffentlichungstag der Anmeldung: 26.11.2008
(73) Patentinhaber: Carl Zeiss Meditec AG, 07745 Jena (DE)
(72) Erfinder: MÜHLHOFF, Dirk, 07751 Jena (DE); WIECHMANN, Martin, 07745 Jena (DE); BISCHOFF, Mark, 07749 Jena (DE); FESTAG, Karsten, 07749 Jena (DE)
(74) Vertreter: Beck, Bernard
(86) Internationale Anmeldenummer: PCT/EP2007/001897
(87) Internationale Veröffentlichungsnummer: WO 2007/104448

(56) Entgegenhaltungen:
- WO-A-97/46184
- DE-U1-202005 018 911
- FR-A- 2 680 677
- US-A- 5 891 132
- US-B1- 7 123 751

## Beschreibung

Die Erfindung bezieht sich auf ein System zur Behandlung oder Diagnose eines Auges eines Patienten, wobei mehrere Behandlungs- oder Diagnosegeräte zeitlich mittelbar oder unmittelbar aufeinander folgend zur Anwendung kommen.

Es ist bekannt, zur Behandlung oder zur Diagnose am Auge einzelne, in ihrer Konzeption dem jeweiligen Anwendungszweck angepaßte Geräte zu benutzen. Soll beispielsweise eine Fehlsichtigkeit durch laserchirurgischen Eingriff an der Cornea des Auges korrigiert werden, so werden zunächst Geräte benutzt, die zur Diagnose am Auge geeignet sind, wie beispielsweise Spaltlampen, optische Anordnungen zur 3D-Messung an der Cornea, Anordnung zur optischen Kohärenztomographie oder ähnlich.

Auf Grundlage der mit einem oder mehreren solcher Diagnosegeräte erzielten Ergebnisse werden nachfolgend die Maßnahmen zur Behandlung festgelegt, zur Behandlung verfügbare Geräte ausgewählt und bereitgehalten.

Bei der Behandlung wird vor dem die Fehlsichtigkeit korrigierenden Eingriff zunächst mittels eines sogenannten "Flapschneide"-Lasergerätes, auch als Laserkeratom oder, in der mechanischen Variante, als Mikrokeratom bekannt, an der Oberfläche der Cornea ein als "Flap" bezeichneter, klappenartiger Deckel ausgebildet, dessen Dicke wesentlich geringer ist als die Dicke der Cornea. Um einen solchen Deckel möglichst schonend und präzise herstellen zu können, werden Laserkeratome benutzt, die einen Behandlungslaserstrahl mit Pulsbreiten kleiner als 10⁻¹²s erzeugen. Damit ist es möglich, in der Cornea lokal begrenzte, eine Ausdehnung von nur wenigen Mikrometern aufweisende Durchbrüche, sogenannte Photodisruptionen, zu erzielen. Durch eine gezielte Aneinanderreihung einer Vielzahl solcher Durchbrüche kann der gewünschte "Flap" ausgebildet und klappbar gestaltet werden.

Unmittelbar auf die Herstellung des "Flap" folgend wird mit einem weiteren Gerät bei weggeklapptem "Flap" von dem so freigelegten inneren Bereich der Cornea zwecks Korrektur der Fehlsichtigkeit Gewebe abgetragen. Diese Abtragung erfolgt wiederum durch Energieeintrag mittels eines gepulsten Behandlungslaserstrahls, der beispielsweise von einem Excimerlaser ausgeht. Derartige zur Abtragung von biologischem Gewebe ausgebildete Geräte werden in der weiteren Beschreibung als Ablationslasergeräte bezeichnet.

Nach der Behandlung werden in der Regel wiederum Diagnosegeräte genutzt, um das Behandlungsergebnis bewerten und gegebenenfalls Nachbehandlungen veranlassen zu können.

Daraus ist ersichtlich, daß die Abfolge der für die Diagnose bzw. therapeutische Behandlung am Auge zur Anwendung kommenden Geräte logistisch durchdacht sein muß, wobei insbesondere patientenbezogene Identifikations-, Diagnose- und/oder Therapiedaten wie auch behandlungsbezogene Konfigurations- oder Protokolldaten sowie Steuersignale in Betracht zu ziehen sind, damit der behandelnde Arzt bzw. der Nutzer an dem jeweils zur Anwendung kommenden Gerät die für die Behandlung oder Diagnose erforderlichen Einstellungen vornehmen kann. Dazu kommt, daß der Patient von Gerät zu Gerät immer neu positioniert werden bzw. das zu behandelnde Auge des Patienten behandlungs- oder diagnosegerecht ausgerichtet werden muß.

Diese Koordination bzw. Abstimmung der Geräte auf die jeweils erforderlichen, auf einen bestimmten Patienten bezogenen Behandlungs- bzw. Diagnoseschritte wird im Stand der Technik insofern erschwert, als die zur Anwendung kommenden Geräte in der Regel in getrennten Räumen aufgestellt sind oder, sofern die Aufstellung innerhalb eines Raumes möglich ist, hinsichtlich ihrer Anwendung zu sehr voneinander entkoppelt sind.

So ist es z.B. erforderlich, die einem Patienten individuell zugeordneten Daten aufzunehmen, daraus Behandlungsdaten zu generieren, diese in das jeweils zur Behandlung vorgesehene Gerät einzugeben und die Behandlungs- und Diagnoseergebnisse anzuzeigen und zu bewerten. Neben der Eingabe der Behandlungsdaten ist auch die Eingabe von Patientendaten, beispielsweise zwecks Identifikation des Patienten, notwendig.

Wegen der Nutzung logistisch voneinander getrennter Geräte im Stand der Technik ist die Behandlung zeitaufwendig und birgt, bedingt durch die vielfach erforderliche Dateneingabe und manuelle Datenübertragung von Gerät zu Gerät, zahlreiche Fehlerquellen.

Weiterhin ist es bekannt, den Patienten auf einer Liege oder einem Stuhl von einem zum nächsten Gerät zu transportierten. Dadurch allein ist jedoch eine präzise Positionierung, die für die Behandlung bzw. Diagnose an dem jeweiligen Gerät erforderlich ist, nicht gewährleistet.

Da die geometrischen Abmessungen der Geräte nicht aufeinander abgestimmt sind, ist es oftmals notwendig, den Patienten nicht nur in einer Ebene von Gerät zu Gerät zu transportieren, sondern auch seine Höhenposition in Abhängigkeit vom jeweiligen Gerätedesign zu verändern. Weiterhin offenbart die WO 97/46184 ein Lasersystem zur Durchführung einer LASIK mit einem drehbaren Patientenbett um dem Patienten einen leichteren Ein- und Ausstieg zu ermöglichen.

Die US 5 891 132 zeigt ein System bestehend aus einem Topographiesystem, einem Computer und einem Excimerlasersystem, welche räumlich getrennt, aber über Datenleitungen miteinander verbunden sind.

Aus der DE 20 2005 018911 U1 ist eine Vorrichtung für die refraktive ophthalmologische Chirurgie mit 2 Behandlungsstationen bekannt, welche eine Liege aufweist, die von einer Behandlungsstation zur anderen verschiebbar ist. Von diesem Stand der Technik ausgehend besteht die der Erfindung zugrunde liegende Aufgabe darin, ein System der eingangs genannten Art zu schaffen, bei dem im Hinblick auf die zeitlich aufeinanderfolgende Anwendung der Geräte ein zügiger Ablauf gewährleistet, Quellen für Behandlungsfehler reduziert und dabei zusätzlich auch die Sicherheit des Patienten vor Verletzungen erhöht ist.
Diese Aufgabe wird mit einem System zur Behandlung oder Diagnose eines Auges eines Patienten gelöst, das in Anspruch 1 definiert ist und das mehrere Geräte zur Behandlung oder zur Diagnose am Auge sowie Mittel zur logistischen Verknüpfung der Geräte miteinander, umfaßt, nämlich
- Vorrichtungen zur räumlichen Positionierung der Geräte relativ zueinander,
- Anordnungen zur Positionierung des Patienten und des zu behandelnden Auges relativ zu den einzelnen Geräten,
- Einrichtungen zur gesteuerten Versorgung der Geräte mit Energie und Hilfsenergie, und/oder
- Mitteilungsmittel zur Übertragung von Informationen oder Steuerbefehlen zwischen den vorgenannten Geräten, Vorrichtungen, Anordnungen bzw. Einrichtungen und zur Ausgabe von Informationen.
In einer Ausführung des erfindungsgemäßen Systems sind mehrere Behandlungs- und/oder Diagnosegeräte vorhanden, von denen in zeitlicher Abfolge jeweils ein vorgeordnetes Gerät vor einem diesem nachgeordneten Gerät zur Anwendung kommt, und bei dem Mitteilungsmittel vorgesehen sind, die - bevorzugt dem jeweils nachgeordneten Gerät - automatisch Informationen darüber übertragen und ausgeben, ob der Patient von dem vorgeordneten Gerät kommend in Bereitschaft zu der am nachgeordneten Gerät beabsichtigten Behandlung oder Diagnose positioniert ist.

Darüber hinaus können die Mitteilungsmittel auch eingesetzt werden, um weitere Informationen oder Steuerbefehle zu übertragen und so Aktionen des nachgeordneten Gerätes zu initiieren.

Diese Mitteilungsmittel können erfindungsgemäß mechanische, akustische, elektronische und/oder opto-elektronische Anordnungen zur Erfassung, Verarbeitung, Übertragung und Ausgabe von auf den Patienten, auf die Ausrichtung des Auges des Patienten und/oder auf die Behandlung oder Diagnose bezogene Daten umfassen. Zur Erfassung können Anordnungen zur manuellen Eingabe von Daten und/oder zur Übernahme von Daten von einem oder mehreren der Geräte in eine Datenverarbeitungseinrichtung oder in Datenspeicher vorhanden sein. Zur Datenausgabe kann das System mit mindestens einer optoelektronischen Anzeigevorrichtung ausgestattet sein.

Dabei können diese Anordnungen ausgebildet sein:
- zur Erfassung, Erzeugung und Verarbeitung patientenbezogener Identifikations-, Diagnose- und/oder Therapiedaten und zu deren Übertragung zwischen den Geräten,
- zur Erfassung und Verarbeitung behandlungsbezogener Konfigurationsdaten, Protokolldaten und/oder Steuersignale und zu deren Übertragung zwischen den Geräten,
- zur Erfassung, Erzeugung, Verarbeitung, Übertragung und Ausgabe von Daten, welche die Sicherheit bei der Ausrichtung des Auges des Patienten relativ zu dem jeweils zur Anwendung vorgesehenen Gerät betreffen.

Zur Behandlung des Auges umfaßt das System in einer ersten Ausgestaltung als vorgeordnetes Gerät ein Laserkeratom zur Erzeugung eines Flapschnittes in der Oberfläche der Cornea des Auges und als nachgeordnetes Gerät ein mit einem Excimer-Laser ausgestattetes Ablationslasergerät zur ablativen Bearbeitung der Cornea.

Alternativ dazu können in einer zweiten Ausgestaltung als vorgeordnetes Gerät ein Mikrokeratom zur Erzeugung des Flapschnittes in der Oberfläche der Cornea des Auges und als nachgeordnetes Gerät ein Ablationslasergerät zur ablativen Bearbeitung der Cornea vorhanden sein.

Neben diesen zur Behandlung ausgebildeten Geräten kann das System weiterhin Geräte zur Diagnose am Auge umfassen, wie beispielsweise Spaltlampen, optische Anordnungen zur 3D-Messung und zur Geometriekontrolle nach Scheimpflug, oder Anordnungen zur optischen Kohärenztomographie (OCT), zur Topographie, zur Wellenfront-Diagnostik oder zur Pachymetrie. Mit diesen Geräten lassen sich die Informationen ermitteln, die vorab zur Festlegung der Behandlungsschritte erforderlich sind.

Auch können die Informationen mit diesen Geräten weiter verarbeitet und dabei mittels entsprechender Software Daten erzeugt werden, die zum Beispiel dazu dienen, das Laserkeratom und/oder das Ablationslasergerät zu steuern.

Werden diese Geräte in das erfindungsgemäße System einbezogen und in der vorbeschriebenen Weise über die genannten Mitteilungsmittel miteinander verknüpft, kann die Behandlung bzw. Diagnose wesentlich zügiger erfolgen als im Stand der Technik und die Quellen für Behandlungsfehler aufgrund mangelhafter Datenübertragung sind wesentlich reduziert.

Um zusätzlich die Sicherheit für den Patienten noch weiter zu erhöhen, sollten in einer weiteren besonders bevorzugten Ausgestaltung des Systems eine Patientenlagerungseinrichtung zum Positionieren des Patienten relativ zu den Geräten und Mittel zur Zwangsführung und Positionsänderung der Patientenlagerungseinrichtung vorhanden sein, wobei
- in einer ersten Vorzugsposition sich das zu behandelnde Auge des Patienten im Behandlungsbereich des vorgeordneten Gerätes zur Erzeugung eines Flapschnittes in der Oberfläche der Cornea befindet,
- in einer zweiten Vorzugsposition sich das zu behandelnde Auge des Patienten im Behandlungsbereich des nachgeordneten Gerätes zur ablativen Bearbeitung der Cornea befindet,
- in einer dritten oder weiteren Vorzugsposition sich das zu behandelnde Auge des Patienten im Meß- oder Beobachtungsbereich eines vor- oder nachgeordneten Gerätes zur Diagnose am Auge befindet, und wobei
- Einrichtungen zur Positionserkennung und/oder zur Arretierung der Patientenlagerungseinrichtung in den Vorzugspositionen als Bestandteile der Mitteilungsmittel vorgesehen sind.

Der Bewegungspfad von einer Vorzugsposition zur nächsten kann prinzipiell beliebig verlaufen, jedoch ist bevorzugt die Positionsänderung durch Bewegung auf einer Kreisbahn vorgesehen, wobei die Vorzugspositionen Positionen auf der Kreisbahn sind, oder es ist die Bewegung auf einer Geraden vorgesehen, wobei die Vorzugspositionen Positionen auf dieser Geraden sind.

Im Rahmen der Erfindung liegt es, wenn mindestens eine Ein- und Ausstiegsposition für den Patienten außerhalb der genannten Vorzugspositionen vorgesehen ist, an der die Patientenlagerungseinrichtung angehalten werden kann, um den Patienten zu Beginn der Diagnose oder Behandlung aufzunehmen und danach zu entlassen.

Die Mittel zur Positionsänderung der Patientenlagerungseinrichtung sollten mindestens einen Positionierantrieb umfassen, der mit einer Positioniersteuerung in Verbindung steht, wobei von der Positioniersteuerung Stellbefehle an den Positionierantrieb gerichtet sind und Vorrichtungen zur Kontrolle und Bestätigung vorgegebener Positionen vorhanden sind.

Die Positioniersteuerung kann dabei ausgerüstet sein mit einem Eingabemodul zur manuellen Vorgabe der Stellbefehle, zum Beispiel in Form eines Joysticks, oder mit einer Regelung zur automatischen Generierung der Stellbefehle anhand der von Meßwertaufnehmern ermittelten Weg- oder Kraftmeßgrößen. Zur Erfassung von Wegmeßgrößen kann ein mit der Positionssteuerung in Verbindung stehendes inkrementell oder absolut messendes Positionsmeßsystem oder ein optisches Positionsmeßsystem mit einem Kameramodul vorhanden sein.

Im Rahmen der Erfindung liegt es weiterhin, wenn die Positioniersteuerung mit einer Vorrichtung zur vollautomatischen Patienten- und Augenpositionierung in Verbindung steht. Diese kann über eine navigationsgestützte Antriebssteuerung verfügen, welche mit Elementen zur Augenpositionserfassungs- und Augenpositionsnachführung ausgestattet ist, die über ein Steuermodul mit den Positionierantrieben in Verbindung stehen.

Diese Antriebssteuerung kann weiterhin mit Sensoren zur Erkennung von Hindernissen versehen sein, die ebenfalls über das Steuermodul mit dem Positionierantrieben in Verbindung stehen. Damit ist die Umgehung von Hindernissen möglich, was insbesondere im Hinblick auf die verschiedenen Gerätegeometrien von Nutzen ist.

Wird zur Behandlung ein auf das Auge aufgesetztes Kontaktglas genutzt, durch das hindurch der Behandlungs-Laserstrahl auf das Auge gerichtet ist, sollte zur Erhöhung der Sicherheit für den Patienten eine Kopfanlage verwendet werden, das Kontaktglas sollte beweglich gelagert sein und es sollte ein Sicherheitsmechanismus vorhanden sein, der über einen Kraft-Meßwertaufnehmer verfügt und durch den die Kopfanlage und das Kontaktglas auseinanderbewegt werden, wenn das Kontaktglas mit übermäßiger Kraft auf das Auge einwirkt. Auf diese Weise wird ein hohes Maß an Sicherheit im Hinblick auf die Vermeidung von Quetschungen am Auge erzielt.

Der Sicherheitsmechanismus sollte das Auseinanderbewegen von Kopfanlage und Kontaktglas erst bei einer oberhalb eines vorgegebenen Grenzwertes wirkenden Kraft veranlassen und bei unterhalb dieses Grenzwertes wirkender Kraft die Relativpositionen von Kopfanlage und Kontaktglas zueinander fixieren.

Das System kann vorteilhaft einen Grundkörper aufweisen, der zur Aufnahme der zum System gehörenden Geräte ausgebildet und mit Haltevorrichtungen zur Befestigung der Geräte ausgestattet ist. Damit ist jedem der Geräte innerhalb des Systems eine Position fest zugeordnet, was vorteilhaft zur Erhöhung der Positioniergenauigkeit des Patienten beiträgt.

Im Rahmen der Erfindung liegt es weiterhin, wenn der Grundkörper mit einer Basis für Zwangsführungen für den Bewegungsablauf bei der Positionsänderung der Patientenlagerungseinrichtung ausgestattet ist. Diese Zwangsführungen können beispielsweise in Form von Leitwegen, wie etwa Schienen, in die Basis eingelassen sein. Die Zwangsführungen können in einer alternativen Ausführungsvariante eine Drehachse zur Führung von Bewegungen der Patientenlagerungseinrichtung auf einer Kreisbahn umfassen.
Dabei hat es sich gezeigt, dass es besonders vorteilhaft ist, wenn die Drehachse die Patientenlagerungseinrichtung nicht mittig, sondern zum Fußende hin verschoben durchdringt.

Die Mitteilungsmittel können Verbindungen zur drahtlosen oder drahtgebunden Übertragung von Daten in Form eines Peer-to-Peer-Netzwerkes umfassen, bevorzugt in Form eines serverbasierten Netzwerkes oder in Form eines Bus-, Stern- oder Ringnetzwerkes. Sofern eine drahtgebunden Übertragung vorgesehen ist, kann das dazu erforderliche Kabelnetz zumindest zu Teilen im Grundkörper untergebracht sein.

Zwecks Versorgung mit Energie und/oder Hilfsenergie sollten die zum System gehörenden Geräte vorzugsweise an eine gemeinsame zentrale Versorgungseinheit gekoppelt sein, und die zentrale Versorgungseinheit sollte mit mindestens einem Notfall-Versorgungsaggregat ausgestattet sein.

Es hat sich gezeigt, dass es für den Patienten angenehm und beruhigend ist, wenn die Umgebung seines Kopfes in der Behandlungsposition indirekt mit einem angenehmen Farbton (z.B. hellblau) beleuchtet ist. Dazu kann das Gehäuse des Behandlungslasers an den entsprechenden Stellen zumindest teilweise durchscheinend und damit "selbstleuchtend" ausgeführt sein.

Die Erfindung soll nachfolgend anhand eines Ausführungsbeispieles näher erläutert werden.

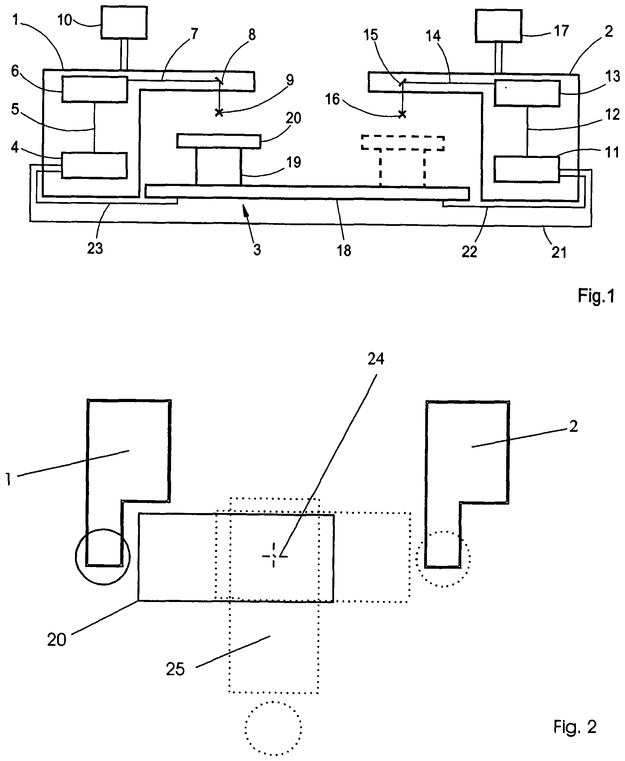

Eine zugehörige Zeichnung zeigt in Fig.1 beispielhaft das Prinzip des erfindungsgemäßen Systems zur Behandlung oder Diagnose eines Auges. Das System umfaßt mehrere Behandlungs- oder Diagnosegeräte, die zeitlich aufeinanderfolgend zur Anwendung kommen. Dabei ist in der Abfolge der Behandlung ein erstes Gerät einem zweiten vorgeordnet bzw. das zweite Gerät dem ersten nachgeordnet.

Zur Erläuterung wird ein Beispiel gewählt, bei dem das System gemäß Fig.1 zunächst ein Laserkeratom 1, ein Ablationslasergerät 2 und eine für diese beiden Geräte gemeinsam genutzte Patientenlagerungseinrichtung 3 umfaßt.

Das Laserkeratom 1 ermöglicht es, unter Nutzung von Femtosekunden-Laserpulsen in einer aus dem Stand der Technik bekannten Weise Schnitte in der Cornea auszuführen und dabei einen "Flap" zu erzeugen. Es beinhaltet eine Steuereinheit 4, welche über eine Steuerleitung 5 unter anderem eine Laserquelle 6 ansteuert. Die Laserquelle 6 emittiert einen Laserstrahl 7, der über einen Scanner 8 und eine Optik (nicht dargestellt) auf ein erstes Behandlungsgebiet 9 gerichtet ist. Die für die Behandlung notwendigen behandlungsbezogenen und patientenbezogenen Daten können über eine Eingabevorrichtung mit einer graphischen Bedienoberfläche 10 eingegeben werden.

Das Ablationslasergerät 2 ermöglicht es, unter Nutzung von UV-Laserstrahlung durch das ebenfalls aus dem Stand der Technik bekannte Photoablationsverfahren Corneagewebe unter dem aufgeklappten "Flap" abzutragen. Es beinhaltet eine Steuereinheit 11, welche über eine Steuerleitung 12 unter anderem eine Laserquelle 13 steuert. Die Laserquelle 13 emittiert einen UV-Laserstrahl 14, der über einen Scanner 15 und eine Optik (nicht dargestellt) auf ein Behandlungsgebiet 16 gerichtet ist. Die für die Behandlung notwendigen behandlungsbezogenen und patientenbezogenen Daten können hier über eine Eingabevorrichtung mit einer graphischen Bedienoberfläche 17 eingegeben werden.

Die Patientenlagerungseinrichtung 3 besteht aus einer unbeweglichen Basis 18, einem Bewegungselement 19 und einer Liegenfläche 20. Die Liegenfläche 20 ist gegenüber dem Bewegungselement 19 in allen drei Raumrichtungen verschiebbar und zu diesem Zweck mit motorischen Antrieben gekoppelt (nicht dargestellt).

Zur manuellen Auslösung und Steuerung der Verschiebung ist ein Joystick (nicht dargestellt) vorgesehen. Alternativ dazu kann vorgesehen sein, daß die Steuereinheit 4 des Laserkeratoms 1 und/oder die Steuereinheit 11 des Ablationslasergerätes 2 Befehle zur Auslösung und Steuerung der Verschiebung generieren und an die motorischen Antriebe ausgeben.

Charakteristisch für diese spezielle Ausführung der Patientenlagerungseinrichtung 3 ist, daß das Bewegungselement 19 beweglich gegenüber der Basis 18 ist und dabei zumindest zwei definierte Vorzugspositionen vorgesehen sind, die mit den Behandlungsgebieten 9 und 16 insofern kommunizieren, als die Ausrichtung der Liegenfläche 20 relativ zum Behandlungsgebiet 9 in einer ersten Vorzugsposition die gleiche ist wie die Ausrichtung der Liegenfläche 20 zum zweiten Behandlungsgebiet 16 in der zweiten Vorzugsposition.

In diesem Zusammenhang umfaßt das erfindungsgemäße System Mitteilungsmittel, die automatisch darüber Auskunft geben, ob das zu behandelnde Auge des Patienten in Bereitschaft zur Behandlung im Behandlungsgebiet 9 des Laserkeratoms 1 positioniert ist (erste Vorzugsposition), sowie automatisch auch darüber Auskunft geben, ob das zu behandelnde Auge des vom Laserkeratom 1 mittels des Bewegungselementes 19 zum Ablationslasergerät 2 transportierten Patienten in Bereitschaft zur nachfolgenden Behandlung im Behandlungsgebiet 16 des Ablationslasergerätes 2 positioniert ist (zweite Vorzugsposition).

Um diese räumliche Beziehung sicherzustellen, kann wahlweise vorgesehen sein, die Bewegung des Bewegungselements 19 und der Liegenfläche 20 relativ zur Basis 18 durch mechanische Anschläge zu begrenzen, oder die Bewegung des Bewegungselementes 19 und der Liegenfläche 20 relativ zur Basis 18 über Einrichtungen zur Positionserfassung und zur Positionssteuerung zu kontrollieren, wobei diese Einrichtungen so programmiert sind, daß die jeweils gewünschte Vorzugsposition erkannt und die Bewegungen des Bewegungselements 19 und der Liegenfläche 20 bei Erreichen dieser Vorzugsposition gestoppt wird.

Mit den definiert festgelegten Vorzugspositionen wird sichergestellt, daß der Patient sowohl dem Laserkeratom 1 als auch dem Ablationslasergerät 2 in Bezug auf die Behandlung sicher und reproduzierbar zugeführt wird. Zudem wird auf diese Weise erreicht, daß der Patient durch eine unkomplizierte Bewegung des Bewegungselementes 19 von der ersten Vorzugsposition in die zweite Vorzugsposition bewegt wird und keine erhebliche manuelle Nachjustierung über Verstellwege von größer 100 mm, zum Beispiel durch Verschiebung der Liegenfläche 20 relativ zum Bewegungselement 19, notwendig ist.

Die Bewegung des Bewegungselementes 19 relativ zur Basis 18 kann dabei auf verschiedene Arten erfolgen.

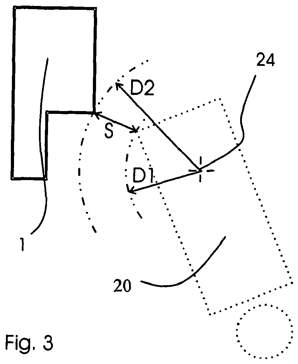

In einer ersten Variante kann das Bewegungselement 19 gegenüber der Basis 18 um eine senkrecht auf der Basis 18 stehenden Achse verdrehbar gelagert sein, so daß sich das Bewegungselement 19 mit der Liegenfläche 20 auf einer Kreisbahn von einer Vorzugsposition in die andere bewegt. Dabei ist die Liegfläche 20 und damit der Kopf, genauer: das zu behandelnde Auge des Patienten, der Abfolge der Behandlung entsprechend zunächst im Behandlungsgebiet 9 des Laserkeratoms 1 und nach Ausführung der Drehbewegung im Behandlungsgebiet 16 des Ablationslasergerätes 2 positioniert. Mit anderen Worten: die beiden Vorzugspositionen sind Positionen auf der Kreisbahn, die das Bewegungselement 19 einschließlich der Liegenfläche 20 bei der Drehbewegung beschreibt.
In einer alternativen Ausgestaltung kann das Bewegungselement 19 ortsfest gestaltet sein und sich die Liegenfläche 20 um eine durch das Bewegungselement 19 definierte Drehachse 24 drehen. Eine solche Variante ist in Fig. 2 in Draufsicht dargestellt. Dabei ist die Drehachse 24 nicht symmetrisch zur Liegenfläche 20 angeordnet, sondern zum Fußende der Liegenfläche 20 verschoben. Diese Lösung ist deshalb von Vorteil, weil das Fußende der Liegenfläche 20 auch bei einer Drehung um einen Winkel größer 90 Grad nicht mit Gehäuseteilen der Behandlungslaser 1 und 2 kollidiert, außerdem bietet die Alternativposition 25 eine bequeme Ein- und Ausstiegsgelegenheit für den Patienten. Fig. 3 zeigt die Platzverhältnisse noch einmal anschaulich. Der Abstand D1 zwischen der Drehachse 24 und einer Ecke des Fußendes der Liegenfläche 20 muss um einen Sicherheitsabstand S kleiner sein als der Abstand D2 der Drehachse 24 von der nächstgelegenen Gehäusekontur des jeweiligen Behandlungslasers 1 (oder 2) damit Kollisionen ausgeschlossen sind.

Alternativ zur Drehbewegung kann in einem Beispiel, das nicht Teil der Erfindung ist, das Bewegungselement 19 in einer zweiten Variante auch translatorisch von der einen zur anderen Vorzugsposition bewegt werden. Hierzu wird das Bewegungselement 19 auf Schienen oder vergleichbaren Führungselementen geführt, die in diesem Fall Teil der Basis 18 sind. Die beiden Vorzugspositionen sind dann ebenfalls Positionen auf der Bahn, die das Bewegungselement 19 einschließlich der Liegenfläche 20 bei der Translation beschreibt.

Im Rahmen weiterer Beispiele, die nicht Teil der Erfindung sind, liegen auch Ausgestaltungen, bei denen das Bewegungselement 19 mit einem separaten Fahrwerk versehen ist und dieses frei von Führungen von einer der Vorzugspositionen in die andere gefahren wird. Dabei geschieht die Erkennung der Vorzugspositionen z.B. durch Sensoren, welche geeignet sind, in die Basis 18 eingelassene oder am Laserkeratom 1 und am Ablationslasergerät 2 angebrachte Marken positionsgenau zu erkennen.

Die Basis 18 kann in beiden vorgenannten Varianten so ausgelegt werden, daß sie sich räumlich einerseits bis zur Grundfläche des Laserkeratoms 1 und andererseits bis zur Grundfläche des Ablationslasergerätes 2 erstreckt. Des weiteren kann die Basis 18 zur Unterbringung von Kabeln ausgestaltet sein, die beispielsweise zum Datenaustausch zwischen dem Laserkeratom 1, dem Ablationslasergerät 2 und der Patientenlagerungseinrichtung 3 dienen, und sie kann in diesem Zusammenhang Kabelein- und Kabelaustritte aufweisen.

In einer weiteren bevorzugten Ausgestaltung des erfindungsgemäßen Systems ist die Basis 18 Bestandteil eines Grundkörpers (nicht dargestellt), auf dem das Laserkeratom 1 und auch das Ablationslasergerät 2 mit ihren Grundflächen aufgestellt und befestigt sind. Diesbezüglich ist es denkbar, Positionsmarkierungen für das Laserkeratom 1 bzw. das Ablationslasergerät 2 oder Fixiervorrichtungen für diese Geräte auf dem Grundkörper vorzusehen.

Weiterhin können die Basis 18 und auch der Grundkörper begehbar ausgelegt sein. Die äußere Umrandung der Basis 18 bzw. des Grundkörpers ist dann entweder vertikal als Stufe ausgeführt, wobei eine Stufenhöhe von 18 cm nicht überschritten werden sollte, oder sie ist schräg als Rampe geformt, um die Stolpergefahr beim Begehen zu minimieren.

Die Oberfläche der Basis 18 bzw. des Grundkörpers sollten den Anforderungen an Fußböden in Operationsräumen entsprechen, um die Rutschgefahr in Grenzen zu halten und auch eine effiziente Reinigung zu ermöglichen.

Unabhängig von den bereits beschriebenen Möglichkeiten der Auslösung und Steuerung der Bewegung des Bewegungselementes 19 kann die Bewegung auf weitere, aus dem Stand der Technik bekannte Weisen manuell oder motorisch ausgelöst oder gesteuert werden. Die manuelle Variante hat dabei den Vorzug, da die die Bewegung steuernde Person unmittelbar darauf achten kann, daß Kollisionen vermieden werden.

Eine motorisch ausgeführte Bewegung hat demgegenüber den Vorteil, daß keine Kraftanstrengungen des bedienenden Personals erforderlich sind. Zudem kann eine solche Bewegung auch aus mehreren Teilbewegungsabläufen zusammengesetzt werden. Insbesondere ist es möglich, den Bewegungsablauf so zu gestalten, daß eine Vorzugsposition verlassen wird, indem zunächst die Liegenfläche 20 abgesenkt wird und erst danach das Bewegungselement 19 gemeinsam mit der Liegenfläche 20 und dem Patienten zu dem Gerät verschoben wird, das nachfolgend zur Behandlung vorgesehen ist. Anschließend wird die Liegenfläche 20 wieder nach oben bewegt, bis die zweite Vorzugsposition eingenommen ist. Durch diese Bewegung auf einer U-förmigen Bahnkurve wird wirkungsvoll vermieden, daß Teile des Laserkeratoms 1 oder des Ablationslasergerätes 2, die in die Behandlungsgebiete 9 bzw. 16 hineinragen, den Patienten verletzen.

Außerdem besteht dadurch die Möglichkeit, das Behandlungsgebiet 9 des Laserkeratoms 1 und das Behandlungsgebiet 16 des Ablationslasergerätes 2 in unterschiedlichen Höhen über der Aufstellfläche bzw. über dem gemeinsamen Grundkörper anzuordnen. In diesem Fall ist es lediglich erforderlich, für die Höhenverstellung der Liegenfläche 20 bei der Positionierung relativ zu beiden Geräten unterschiedliche senkrechte Verfahrwege vorzugeben.

Dagegen ist es selbstverständlich vorteilhaft, wenn die Geräte zumindest soweit baugleich ausgeführt sind, daß die Behandlungsgebiete 9 und 16 etwa die gleiche Höhe über einer gemeinsamen Aufstellebene haben, wodurch sichergestellt ist, daß die Liegenfläche 20 mit dem Patienten während der Positionierbewegung nicht in der Höhe verfahren werden muß. Neben der Zeitersparnis führt dies vor allem zu einem erhöhten Maß an Sicherheit.

Zur Erhöhung des Patientenkomforts weisen die Gehäuse eines oder beider Behandlungslaser 1, 2 in der Nähe der Behandlungsgebiete 9, 16 eine indirekte Beleuchtung, z.B. durch transluzente Ausführung von Gehäuseteilen, auf, mit deren Hilfe farbiges Licht, welches als angenehm empfunden wird, erzeugt wird.

Darüber hinaus weist das System spezifische Sicherheitsfunktionen auf, die insbesondere der Vermeidung von Quetschungen insbesondere am Auge des Patienten dienen.

Zu diesem Zweck ist die Patientenlagerungseinrichtung 3 mit einer Positionserkennung ausgestattet (nicht dargestellt). Die Positionserkennung verfügt zur Erfassung von Wegmeßgrößen über ein mit einer Positioniersteuerung in Verbindung stehendes inkrementell oder absolut messendes Positionsmeßsystem oder über ein optisches Positionsmeßsystem mit einem Kameramodul.

Die Positioniersteuerung ist in diesem Fall ihrerseits mit einer Vorrichtung zur vollautomatischen Patienten- und Augenpositionierung verbunden, die über eine navigationsgestützte Antriebssteuerung verfügt und die mit Elementen zur Augenpositionserfassungs- und Augenpositionsnachführung sowie mit Sensoren zur Erfassung von Hindernissen und einem mit diesen Sensoren und den Positionierantrieben in Verbindung stehenden Steuermodul zur Umgehung der Hindernisse ausgestattet ist.

Damit sind stets Informationen darüber verfügbar, in welcher Position sich die Patientenlagerungseinrichtung 3 befindet, insbesondere ob sie sich in einer der Vorzugspositionen befindet, beispielsweise ob der Patient von einem vorgeordneten Gerät kommend am nachgeordneten Gerät in Bereitschaft zu der dort beabsichtigten Behandlung positioniert ist, oder ob sie sich dazwischen befindet. Die Genauigkeit der Positionserkennung beträgt wenigstens etwa 100 mm, im Idealfall jedoch nur etwa 100 *µ*m. Abhängig von der Position und abhängig von weiteren Signalen, welche die Positioniersteuerung von einem der Geräte oder von beiden Geräten empfängt, werden bestimmte Bewegungen der Patientenlagerungseinrichtung blockiert, um Kollisionen beim Bewegungsablauf zu vermeiden.

Beispielsweise können so alle unerwünschten Bewegungen der Liegenfläche 20 und des Bewegungselements 19 blockiert werden, wenn im Behandlungsgebiet 9 des Laserkeratoms 1 auf das Auge des Patienten ein Kontaktglas aufgesetzt ist, durch das hindurch der Behandlungs-Laserstrahl des Laserkeratoms 1 auf das Auge gerichtet wird, so daß diesbezüglich verursachte Quetschungen am Auge ausgeschlossen sind.

Eine besonders vorteilhafte und einfache Ausführung besteht darin, daß die Positionserkennung zwei Schalter aufweist, welche in der ersten Vorzugsposition eine erste Teilmenge von Interlockfunktionen aktivieren und in der zweiten Vorzugsposition eine zweite Teilmenge von Interlockfunktionen aktivieren. Dabei wird die erste Teilmenge der Interlockfunktionen durch das Laserkeratom 1 gesteuert und die zweite Teilmenge durch das Ablationslasergerät 2.

Um einen besonders flüssigen Ablauf bei der Behandlung oder Diagnose zu gewährleisten, kann vorteilhaft eine Datenverbindung 21 zwischen dem Laserkeratom 1 und dem Ablationslasergerät 2 vorgesehen sein. Die Datenverbindung 21 kann drahtlos oder drahtgebunden ausgeführt sein und muß nicht als direkte Verbindung zwischen den Geräten bestehen, sondern kann auch über zwischengeschaltete Datenverarbeitungsgeräte oder Datenspeicher vermittelt werden. Die Datenverbindung 21 hat beispielsweise die Aufgabe, patientenbezogene Daten zwischen den Geräten abzugleichen, die Steuerung der Geräte hinsichtlich der Behandlungsparameter zu synchronisieren und/oder Diagnoseergebnisse, die in Behandlungsparameter einfließen sollen, zu übermitteln.

So enthält ein patientenbezogener Datensatz beispielsweise folgende Daten:
- Patientenname und Identifikation (Geburtsdatum, Aktennummer usw.),
- Identifikation des Auges,
- Art der Fehlsichtigkeit,
- behandlungsbezogene Korrekturzielwerte,
- Durchmesser der zu behandelnden optischen Zone,
- Flapdurchmesser, Flapdicke, Schnittführungsparameter des Flaprandes,
- Datum der Behandlung.

Diese Daten werden zunächst über eine Eingabemaske an zentraler Stelle oder über die Bedienoberflächen 10, 17 an den Geräte eingegeben. Aufgabe der Datenverbindung ist es nun, diese Daten vollständig oder teilweise an das jeweils andere Gerät zu übertragen, damit sie dort für die nachfolgende Behandlung bereitstehen. Auf diese Weise werden Fehlzuordnungen von Behandlungsdaten zu Patientendaten vermieden.

Werden die Daten nach der Eingabe und Übertragung noch an einem der beiden Geräte geändert, so ist es Aufgabe der Datenverbindung, die Änderungen zu übertragen und so dafür zu sorgen, daß der Datensatz auf beiden Geräten stets aufeinander abgestimmt und in der Regel identisch ist.

Wie bereits dargestellt, kann die Datenverbindung genutzt werden, um die Steuerungsaktionen zwischen dem Laserkeratom 1 und dem Ablationslasergerät 2 zu synchronisieren, so daß während der Behandlung Aktionen des Nutzers am einen Gerät Aktionen am jeweils anderen Gerät bewirken. Dazu wird im folgenden beispielhaft ein möglicher Behandlungsablauf beschrieben:

Zu Beginn der Behandlung stehen die Patientendaten vollständig auf beiden Geräten zur Verfügung. Mit Hilfe der graphischen Bedienoberfläche 10 am Laserkeratom 1 wird aus einer Datenbank der Patientendatensatz ausgewählt, der dem zu behandelnden Patient zugeordnet ist. Die Behandlungsdaten für den "Flap" und die nachfolgende Ablation werden dem Nutzer angezeigt. Er kann von dieser Stelle aus alle Eingaben überprüfen und noch bis zum Beginn der Behandlung verändern. Werden zu diesem Zeitpunkt Werte verändert, welche Einfluß auf die nachfolgende Behandlung mit dem Ablationslasergerät 2 haben, so werden die Änderungen über die Datenverbindung an das Ablationslasergerät 2 übertragen und stehen dort für die auf die Ausführung des Flapschnittes folgende Ablation zur Verfügung.

Bevor jedoch mit der Ausführung des Flapschnittes mit dem Laserkeratom 1 begonnen wird, sendet die Steuereinheit 11 des Laserkeratoms 1 ein Kommando an das Ablationslasergerät 2, woraufhin das Ablationslasergerät 2 einen Selbsttest durchführt und die Laserquelle entsprechend der Behandlungsparameter kalibriert. Erst nachdem das Ablationslasergerät 2 seine Bereitschaft für eine anschließende Ablation per Datenverbindung an das Laserkeratom 1 übermittelt hat, erteilt die Steuereinheit 11 des Laserkeratoms 1 die Freigabe zum Beginn der Schnittprozedur.

Mit dieser Maßnahme wird sichergestellt, daß ein Patient nach Ausführung des Flapschnittes ohne Zeitverzug mit dem Ablationslasergerät 2 weiterbehandelt werden kann. Dadurch wird insbesondere eine unnötige Austrocknung der Cornea verhindert. Zudem wird das Risiko der nicht möglichen Weiterbehandlung eines Patienten wegen Versagens des Ablationslasergerätes 2 erheblich reduziert.

Nachdem die Freigabe erfolgt ist und auch alle Daten und Parameter durch den Nutzer überprüft und bestätigt worden sind, wird nun mit dem Laserkeratom 1 der Flapschnitt an dem zu behandelnden Auge ausgeführt. Nach Abschluß der Schnittprozedur wird die Patientenlagerungseinrichtung 3 mit dem Patienten von der einen Vorzugsposition, bei der sich zu behandelnde das Augen noch im Behandlungsbereich des Laserkeratoms 1 befindet, in die andere Vorzugsposition bewegt, so daß das Auge nun im Behandlungsbereich des Ablationslasergerätes 2 positioniert ist. Zur Ansteuerung der Patientenlagerungseinrichtung 3 sind Datenverbindungen 22 und 23 vorhanden.

Gleichzeitig werden Daten von der Steuerung des Laserkeratoms 1 an das Ablationslasergerät 2 gesendet, welche die Informationen zum Patienten enthalten, der soeben einem Flapschnitt unterzogen wurde. Daraufhin erscheint auf der graphischen Bedienoberfläche 17 des Ablationslasergerätes 2 ein Fenster mit den Daten des Patienten bereits unter Berücksichtigung möglicherweise am Laserkeratom 1 vorgenommener Änderungen sowie die am Laserkeratom 1 ausgewählte Flaptiefe. Der Nutzer muß diese Daten nur noch bestätigen oder gegebenenfalls anpassen und die Ablation kann gestartet werden.

Nach dem Ende der Ablation wird der Patient mittels der Patientenlagerungseinrichtung 3 entweder in eine dritte Vorzugsposition bewegt, die einer Ein- und Ausstiegsposition für den Patienten entspricht, und gebeten aufzustehen oder er wird wieder in die erste Vorzugsposition bewegt um die Therapieprozedur nun mit dem noch nicht behandelten zweiten Auge durchzuführen. Ist Letzteres der Fall, so erscheinen nun auf der graphischen Bedienoberfläche 17 des Laserkeratoms 1 automatisch die Behandlungsdaten für das zweite Auge.

Das bisher beispielhaft beschriebene System aus dem Laserkeratom 1, dem Ablationslasergerät 2 und der Patientenlagerungseinrichtung 3 kann durch weitere Geräte ergänzt werden, insbesondere durch Diagnosegeräte zur Gewinnung der für die Behandlung mit den genannten Behandlungsgeräten erforderlichen Daten oder zur Kontrolle der mit den Behandlungsgeräten erzielten Veränderungen. Dabei kann es sich um Diagnosegeräte handeln, die ausgebildet sind als Spaltlampen, als optische Anordnungen zur 3D-Messung und zur Geometriekontrolle nach Scheimpflug oder auch als Anordnungen zur optischen Kohärenztomographie (OCT), zur Topographie, zur Wellenfront-Diagnostik oder zur Dickenmessung nach dem Prinzip der Pachymetrie.

In die Erfindung eingeschlossen sind darüber hinaus auch Ausgestaltungen des Systems, bei denen eine Datenfernübertragung, beispielsweise über ein Modem, vorgesehen ist. Des weiteren können Computer zur Planung, Steuerung und/oder Speicherung von Behandlungsprozeßdaten vorhanden sein in Form von Planungs- und Diagnose-Einheiten.

Wie bereits dargelegt, können die Verbindungen zur Übertragung der Daten zwischen den Geräten, der Patientenlagerungseinrichtung, den Datenein- und Datenausgabegeräten sowie den Einrichtungen zu Datenverarbeitung drahtlos oder drahtgebunden in Form eines Peer-to-Peer-Netzwerkes ausgeführt sein. Das Netzwerk und die Übertragungsprotokolle sollten so beschaffen sein, daß die Gefahr von Datenverlusten oder Übertragungsfehlern möglichst gering ist, wobei die Verwendung eines CAN-Bus aufgrund der hohen Übertragungssicherheit der Daten zu bevorzugen ist.

### Bezugszeichenliste

- 1: Laserkeratom
- 2: Ablationslasergerät
- 3: Patientenlagerungseinrichtung
- 4: Steuereinheit
- 5: Steuerleitung
- 6: Laserquelle
- 7: Laserstrahl
- 8: Scanner
- 9: Behandlungsgebiet
- 10: Bedienoberfläche
- 11: Steuereinheit
- 12: Steuerleitung
- 13: Laserquelle
- 14: UV-Laserstrahl
- 15: Scanner
- 16: Behandlungsgebiet
- 17: Bedienoberfläche
- 18: Basis
- 19: Bewegungselement
- 20: Liegenfläche
- 21, 22, 23: Datenverbindungen
- 24: Drehachse der Liegenfläche
- 25: Alternativposition

## Patentansprüche

1. System zur Behandlung und/oder Diagnose eines Auges eines Patienten, umfassend
- mehrere Geräte (1, 2) zur Behandlung oder zur Diagnose am Auge, sowie
- Mittel zur logistischen Verknüpfung der Geräte miteinander, nämlich
- Vorrichtungen zur räumlichen Positionierung der Geräte relativ zueinander,
- Anordnungen (3) zur Positionierung des Patienten und des zu behandelnden Auges relativ zu den einzelnen Geräten,
- Einrichtungen zur gesteuerten Versorgung der Geräte mit Energie und Hilfsenergie, und/oder
- Mitteilungsmittel zur Übertragung von Informationen oder Steuerbefehlen zwischen den vorgenannten Geräten, Vorrichtungen, Anordnungen bzw. Einrichtungen und zur Ausgabe von Informationen
wobei ein gemeinsamer Grundkörper (18) zur Aufnahme der Geräte sowie Haltevorrichtungen zur Befestigung der Geräte an dem Grundkörper vorgesehen sind,
bei dem
- zum Positionieren des Patienten relativ zu den Geräten eine Patientenlagerungseinrichtung (3) für den Patienten und Mittel zur Zwangsführung und Positionsänderung (19) der Patientenlagerungseinrichtung (3) vorhanden sind, wobei
- in einer ersten Vorzugsposition sich das zu behandelnde Auge des Patienten im Behandlungsbereich eines vorgeordneten Gerätes (1) zur Erzeugung eines Flapschnittes in der Oberfläche der Cornea befindet,
- in einer zweiten Vorzugsposition sich das zu behandelnde Auge des Patienten im Behandlungsbereich eines nachgeordneten Gerätes (2) zur ablativen Bearbeitung der Cornea befindet,
- und wobei Einrichtungen zur Positionserkennung und/oder zur Arretierung der Patientenlagerungseinrichtung (3) in den Vorzugspositionen als Bestandteile der Mitteilungsmittel vorgesehen sind
**gekennzeichnet dadurch, dass** die Positionsänderung
- durch Bewegung auf einer Kreisbahn erfolgt, wobei die Vorzugspositionen Positionen auf der Kreisbahn sind, oder
- durch Drehbewegung der Patientenlagerungseinrichtung (3) um eine Drehachse (24), welche zwischen Mitte und Fußende der Patientenlagerungseinrichtung (3) angeordnet ist, erfolgt.

2. System nach Anspruch 1, bei dem
- mehrere Behandlungs- oder Diagnosegeräte vorhanden sind, von denen in zeitlicher Abfolge jeweils ein vorgeordnetes Gerät vor einem diesem nachgeordneten Gerät zur Anwendung kommt, und bei dem
- Mitteilungsmittel vorgesehen sind, die automatisch Informationen darüber übertragen und ausgeben, ob der Patient von dem vorgeordneten Gerät kommend in Bereitschaft zu der am nachgeordneten Gerät beabsichtigten Behandlung oder Diagnose positioniert ist.

3. System nach Anspruch 1 oder 2, bei dem die Mitteilungsmittel mechanische, elektronische und/oder opto-elektronische Anordnungen zur Erfassung, Verarbeitung, Übertragung und Ausgabe von auf den Patienten, auf die Ausrichtung des Auges des Patienten und/oder auf die Behandlung oder Diagnose bezogene Daten umfassen.

4. System nach Anspruch 3, bei dem Anordnungen zur Erfassung und Verarbeitung patientenbezogener Identifikations-, Diagnose- und/oder Therapiedaten und zu deren Übertragung zwischen den Geräten vorhanden sind.

5. System nach Anspruch 3, bei dem Anordnungen zur Erfassung und Verarbeitung behandlungsbezogener Konfigurationsdaten, Protokolldaten und/oder Steuersignale und zu deren Übertragung zwischen den Geräten vorhanden sind.

6. System nach Anspruch 3, bei dem Anordnungen zur Erfassung, Verarbeitung, Übertragung und Ausgabe von Daten vorhanden sind, welche die Sicherheit bei der Ausrichtung des Auges des Patienten relativ zu dem jeweils zur Anwendung vorgesehenen Gerät betreffen.

7. System nach einem der vorgenannten Ansprüche, bei dem die Mitteilungsmittel
- mindestens eine Anordnung zur manuellen Eingabe von Daten und/oder zur Übernahme von Daten von einem oder mehreren der Geräte in eine Datenverarbeitungseinrichtung oder in Datenspeicher, sowie
- mindestens eine opto-elektronische Anzeigevorrichtung umfassen.

8. System nach einem der vorgenannten Ansprüche, bei dem
- als vorgeordnetes Gerät ein Femtosekunden-Lasergerät zur Erzeugung eines Flapschnittes in der Oberfläche der Cornea des Auges, und
- als nachgeordnetes Gerät ein mit einem Excimer-Laser ausgestattetes Gerät zur ablativen Bearbeitung der Cornea vorhanden sind.

9. System nach einem der Ansprüche 1 bis 7, bei dem
- als vorgeordnetes Gerät ein als Laserkeratom (1) ausgebildetes Gerät zur Erzeugung eines Flapschnittes in der Oberfläche der Cornea des Auges, und
- als nachgeordnetes Gerät ein mit einem im UV-Bereich arbeitenden Festkörperlaser ausgestattetes Gerät zur ablativen Bearbeitung der Cornea vorhanden sind.

10. System nach einem der vorgenannten Ansprüche mit vor- oder nachgeordneten Geräten zur Diagnose am Auge, die ausgebildet sind als
- Spaltlampen,
- optische Anordnungen zur 3D-Messung und zur Geometriekontrolle nach Scheimpflug, oder als
- Anordnungen zur optischen Kohärenztomographie (OCT), zur Topographie, zur Wellenfront-Diagnostik oder zur Pachymetrie.

11. System nach einem der vorgenannten Ansprüche, bei dem
- in einer dritten oder weiteren Vorzugsposition sich das zu behandelnde Auge des Patienten im Meß- oder Beobachtungsbereich eines vor- oder nachgeordneten Gerätes zur Diagnose am Auge befindet.

12. System nach einem der vorigen Ansprüche, **gekennzeichnet durch** mindestens eine außerhalb der Vorzugspositionen vorgesehene Ein- und Ausstiegsposition für den Patienten.

13. System nach einem der vorigen Ansprüche, bei dem
- die Mittel zur Positionsänderung der Patientenlagerungseinrichtung (3) mindestens einen Positionierantrieb aufweisen,
- der Positionierantrieb mit einer Positioniersteuerung in Verbindung steht,
- von der Positioniersteuerung Stellbefehle an den Positionierantrieb gerichtet sind, und
- Vorrichtungen zur Kontrolle und Bestätigung vorgegebener Positionen vorhanden sind.

14. System nach Anspruch 13, bei dem die Positioniersteuerung ausgerüstet ist mit
- einem Eingabemodul zur manuellen Vorgabe der Stellbefehle, bevorzugt einem Joystick, oder
- einer Regelung zur automatischen Generierung der Stellbefehle anhand der von Meßwertaufnehmern ermittelten Weg- oder Kraftmeßgrößen.

15. System nach Anspruch 14, **dadurch gekennzeichnet, daß** zur Erfassung von Wegmeßgrößen ein mit der Positioniersteuerung in Verbindung stehendes inkrementell oder absolut messendes Positionsmeßsystem oder ein optisches Positionsmeßsystem mit einem Kameramodul vorhanden ist.

16. System nach einem der Ansprüche 12 bis 15, bei dem die Positioniersteuerung mit einer Vorrichtung zur vollautomatischen Patienten- und Augenpositionierung in Verbindung steht, welche über eine navigationsgestützte Antriebssteuerung verfügt, die mit Elementen zur Augenpositionserfassungs- und Augenpositionsnachführung sowie mit Sensoren zur Erfassung von Hindernissen und einem mit diesen Sensoren und den Positionierantrieben in Verbindung stehenden Steuermodul zur Umgehung von Hindernissen ausgestattet ist.

17. System nach einem der Ansprüche 10 bis 16, bei dem zum Positionieren des Patienten vorhanden sind:
- eine Kopfanlage für den Patienten und ein auf das Auge aufsetzbares, beweglich gehaltenes Kontaktglas, durch das hindurch der Behandlungs-Laserstrahl auf das Auge gerichtet ist,
- ein Meßwertaufnehmer für die Kraft, mit der das Kontaktglas auf dem Auge aufsitzt, und
- ein Sicherheitsmechanismus, der über einen Kraft-Meßwertaufnehmer verfügt und durch den die Kopfanlage und das Kontaktglas auseinanderbewegt werden, wenn das Kontaktglas mit übermäßiger Kraft auf das Auge einwirkt.

18. System nach Anspruch 17, **dadurch gekennzeichnet, daß** der Sicherheitsmechanismus das Auseinanderbewegen erst bei einer oberhalb eines Grenzwertes wirkenden Kraft veranlaßt und bei unterhalb dieses Grenzwertes wirkender Kraft die Relativpositionen von Kopfanlage und Kontaktglas zueinander fixiert sind.

19. System nach Anspruch 1, bei welchem der Grundkörper mit einer Basis (18) für Zwangsführungen für den Bewegungsablauf bei der Positionsänderung der Patientenlagerungseinrichtung (3) relativ zu den Geräten ausgestattet ist.

20. System nach Anspruch 19, bei dem die Zwangsführungen in Form von Leitwegen zur Zwangsführung, bevorzugt als Schienen, in die Basis (18) eingelassen sind.

21. System nach einem der vorgenannten Ansprüche, bei dem die Mitteilungsmittel Verbindungen zur drahtlosen oder drahtgebunden Übertragung von Daten in Form eines Peer-to-Peer-Netzwerkes, bevorzugt in Form eines serverbasierten Netzwerkes oder in Form eines Bus-, Stern- oder Ringnetzwerkes, umfassen.

22. System nach Anspruch 21, bei welchem die Übertragung der Daten drahtgebunden erfolgt und Teile des dazu vorgesehenen Netzwerkes im Grundkörper untergebracht sind.

23. System nach einem der vorgenannten Ansprüche, bei dem die Geräte zwecks Energieversorgung an eine zentrale Versorgungseinheit gekoppelt sind.

24. System nach Anspruch 23, bei dem die zentrale Versorgungseinheit mit mindestens einem Notfall-Energieversorgungsaggregat ausgestattet ist.

## Claims

1. System for treating and/or diagnosing an eye of a patient, comprising
- a plurality of appliances (1, 2) for treatment or diagnosis on the eye, and
- means for logistically linking the appliances to one another, namely
- apparatuses for spatial positioning of the appliances relative to one another,
- arrangements (3) for positioning the patient and the eye to be treated relative to the individual appliances,
- devices for controlled supply of the appliances with energy and auxiliary energy, and/or
- communication means for transferring information or control commands between the aforementioned appliances, apparatuses, arrangements and devices, and for outputting information,
wherein a common main body (18) for receiving the appliances and holding apparatuses for fastening the appliances to the main body are provided,
wherein
- a patient-bearing device (3) for the patient and means for forced guidance and positional changes (19) of the patient-bearing device (3) are present for positioning the patient relative to the appliances, wherein
- the eye of the patient to be treated is situated, in a first preferred position, in the treatment range of an upstream appliance (1) for producing a flap incision in the surface of the cornea,
- the eye of the patient to be treated is situated, in a second preferred position, in the treatment range of a downstream appliance (2) for processing the cornea by ablation,
- and wherein devices for recognizing the position of and/or stopping the patient-bearing device (3) at the preferred positions are provided as components of the communication means,
**characterized in that** the positional change
- is brought about by movement along a circular path, wherein the preferred positions are positions on the circular path, or
- by a rotational movement of the patient-bearing device (3) about an axis of rotation (24) which is arranged between the centre and foot end of the patient-bearing device (3).

2. System according to Claim 1, wherein
- a plurality of treatment or diagnosis appliances are present, of which, in a temporal sequence, an upstream appliance is used in each case before an appliance disposed downstream thereof, and wherein
- communication means are provided, which automatically transfer and output information in respect of whether the patient, coming from the upstream appliance, is positioned ready for the treatment or diagnosis intended at the downstream appliance.

3. System according to Claim 1 or 2, wherein the communication means comprise mechanical, electronic and/or optoelectronic arrangements for capturing, processing, transferring and outputting data relating to the patient, relating to the alignment of the eye of the patient and/or relating to the treatment or diagnosis.

4. System according to Claim 3, wherein arrangements are present for capturing and processing patient-related identification, diagnosis and/or therapy data and for transferring these between the appliances.

5. System according to Claim 3, wherein arrangements are present for capturing and processing treatment-related configuration data, protocol data and/or control signals and for transferring these between the appliances.

6. System according to Claim 3, wherein arrangements for capturing, processing, transferring and outputting data are present, which relate to the safety when aligning the eye of the patient relative to the respective appliance provided for application.

7. System according to any one of the preceding claims, wherein the communication means
- comprise at least one arrangement for the manual entry of data and/or for transferring data from one or more of the appliances into a data processing device or data memory, and
- at least one optoelectronic indication apparatus.

8. System according to any one of the preceding claims, wherein
- a femtosecond laser appliance for producing a flap incision in the surface of the cornea of the eye is present as upstream appliance and
- an appliance equipped with an excimer laser for ablative processing of the cornea is present as downstream appliance.

9. System according to any one of Claims 1 to 7, wherein
- an appliance embodied as a laser keratome (1) for producing a flap incision in the surface of the cornea of the eye is present as upstream appliance,
- an appliance equipped with a solid-state laser operating in the UV range for ablative processing of the cornea is present as downstream appliance.

10. System according to any one of the preceding claims, comprising upstream or downstream appliances for a diagnosis on the eye, said appliances being embodied as
- slit lamps,
- optical arrangements for 3D measurements and for monitoring the geometry according to Scheimpflug, or as
- arrangements for optical coherence tomography (OCT), for topography, for wavefront diagnostics or for pachymetry.

11. System according to any one of the preceding claims, wherein
- the eye of the patient to be treated is situated, in a third or further preferred position, in the measurement or observation range of an upstream or downstream appliance for a diagnosis on the eye.

12. System according to any one of the preceding claims, **characterized by** at least one entry and exit position for the patient, provided away from the preferred positions.

13. System according to any one of the preceding claims, wherein
- the means for positional change of the patient-bearing device (3) comprise at least one positioning drive,
- the positioning drive is connected to a positioning controller,
- actuating commands are directed to the positioning drive from the positioning controller, and
- apparatuses for monitoring and confirming predetermined positions are present.

14. System according to Claim 13, wherein the positioning controller is equipped with
- an input module for manual predetermination of the actuating commands, preferably a joystick, or
- a closed-loop control for automatically generating the actuating commands on the basis of the travel or force measurement variables ascertained by measurement value recorders.

15. System according to Claim 14, **characterized in that** an incrementally or absolutely measuring position measurement system connected to the positioning controller or an optical position measurement system comprising a camera module is present for capturing travel measurement variables.

16. System according to any one of Claims 12 to 15, wherein the positioning controller is connected to an apparatus for fully automatic patient and eye positioning, which comprises a navigation-assisted drive controller, which is equipped with elements for eye position capture and eye position update and with sensors for capturing obstacles, and a control module connected to these sensors and the positioning drives for evading obstacles.

17. System according to any one of Claims 10 to 16, wherein the following are present for positioning the patient:
- a headrest for the patient and a movably held contact glass, which may be placed onto the eye and through which the treatment laser beam is directed to the eye,
- a measurement value recorder for the force with which the contact glass sits on the eye, and
- a safety mechanism which is equipped with a force measurement value recorder and by means of which the headrest and the contact glass are moved apart if the contact glass exerts excessive force onto the eye.

18. System according to Claim 17, **characterized in that** the safety mechanism causes the movement apart only in the case of an acting force above a threshold and the relative positions of headrest and contact glass in relation to one another are fixed in the case of an acting force below this threshold.

19. System according to Claim 1, wherein the main body is equipped with a base (18) for carrying out instances of forced guidance for the movement sequence when changing the position of the patient-bearing device (3) relative to the appliances.

20. System according to Claim 19, wherein the instances of forced guidance are embedded into the base (18) in the form of the guiding paths for forced guidance, preferably as rails.

21. System according to any one of the preceding claims, wherein the communication means comprise connections for wireless or wired transfer of data in the form of a peer-to-peer network, preferably in the form of a server-based network, or in the form of a bus network, star network or ring network.

22. System according to Claim 21, wherein the transfer of the data is carried out in a wired manner and parts of the network provided to this end are housed in the main body.

23. System according to any one of the preceding claims, wherein the appliances are coupled to a central supply unit for power supply purposes.

24. System according to Claim 23, wherein the central supply unit is equipped with at least one emergency power supply aggregate.

## Revendications

1. Système de traitement et/ou de diagnostic d'un oeil d'un patient, comprenant
- plusieurs appareils (1, 2) destinés au traitement ou au diagnostic de l'oeil, ainsi que
- des moyens destinés à établir une combinaison logistique entre les appareils, à savoir
- des dispositifs destinés à positionner les appareils dans l'espace les uns par rapport aux autres,
- des ensembles (3) destinés à positionner le patient et l'oeil à traiter par rapport aux différents appareils,
- des installations destinées à alimenter de manière commandée les appareils en énergie auxiliaire, et/ou
- des moyens de communication destinés à la transmission d'informations ou d'instructions de commande entre les appareils, les dispositifs, les ensembles ou les installations précités et à délivrer des informations,
dans lequel il est prévu un corps de base commun (18) destiné à recevoir les appareils ainsi que des dispositifs de maintien permettant de fixer les appareils sur le corps de base,
dans lequel,
- pour le positionnement du patient par rapport aux appareils, il est prévu une installation de mise en place du patient (3) destinée au patient et des moyens destinés au guidage contraint et à la modification de position (19) de l'installation de mise en place du patient (3), dans lequel
- à une première position préférentielle, l'oeil à traiter du patient se trouve dans la zone de traitement d'un appareil amont (1) destiné à produire une incision de rabat dans la surface de la cornée,
- à une deuxième position préférentielle, l'oeil à traiter du patient se trouve dans la zone de traitement d'un appareil aval (2) destiné à effectuer un traitement ablatif de la cornée,
- et dans lequel il est prévu des installations destinées à détecter la position et/ou destinées à bloquer l'installation de mise en place du patient (3) aux positions préférentielles en tant que parties constitutives des moyens de communication,
**caractérisé en ce que** la modification de position est effectuée
- par déplacement sur un trajet circulaire, dans lequel les positions préférentielles sont des positions situées sur le trajet circulaire, où
- par déplacement en rotation de l'installation de mise en place du patient (3) autour d'un axe de rotation (24) qui est disposé entre le centre et l'extrémité inférieure de l'installation de mise en place du patient (3).

2. Système selon la revendication 1, dans lequel
- il est prévu plusieurs appareils de traitement et de diagnostic, parmi lesquels un appareil amont respectif est mis en oeuvre avant un appareil situé en aval par rapport à celui-ci selon une séquence temporelle, et dans lequel
- il est prévu des moyens de transmission qui transmettent et délivrent automatiquement des informations indiquant si le patient provenant de l'appareil amont est positionné de manière à être prêt pour le traitement ou le diagnostic prévu sur l'appareil aval.

3. Système selon la revendication 1 ou 2, dans lequel les moyens de communication comprennent des ensembles mécaniques, électroniques et/ou optoélectroniques permettant de détecter, de traiter, de transmettre et de délivrer des données concernant le patient, l'orientation de l'oeil du patient et/ou le traitement ou le diagnostic.

4. Système selon la revendication 3, dans lequel il est prévu des ensembles destinés à détecter et à traiter des données d'identification, de diagnostic et/ou de thérapie et à les transmettre entre les appareils.

5. Système selon la revendication 3, dans lequel il est prévu des ensembles destinés à détecter et à traiter des données de configuration concernant le traitement, des données de protocole et/ou des signaux de commande et à les transmettre entre les appareils.

6. Système selon la revendication 3, dans lequel il est prévu des ensembles destinés à détecter, à traiter, à transmettre et à délivrer des données qui concernent la sécurité du point de vue de l'orientation de l'oeil du patient par rapport à l'appareil devant respectivement être utilisé.

7. Système selon l'une quelconque des revendications précédentes, dans lequel les moyens de communication comprennent
- au moins un ensemble destiné à saisir manuellement des données et/ou à récupérer des données provenant d'un ou plusieurs des appareils dans une installation de traitement de données ou dans une mémoire de données, et
- au moins un dispositif d'affichage optoélectronique.

8. Système selon l'une quelconque des revendications précédentes, dans lequel il est prévu,
- en tant qu'appareil amont, un appareil à laser femtoseconde destiné à produire une incision de rabat dans la surface de la cornée de l'oeil, et
- en tant qu'appareil aval, un appareil équipé d'un laser excimère destiné à effectuer un traitement ablatif de la cornée.

9. Système selon l'une quelconque des revendications 1 à 7, dans lequel il est prévu,
- en tant qu'appareil amont, un appareil réalisé sous la forme d'un couteau lancéolaire à laser (1) destiné à produire une incision de rabat dans la surface de la cornée de l'oeil, et
- en tant qu'appareil aval, un appareil équipé d'un laser à solide fonctionnant dans le domaine UV et destiné à effectuer un traitement ablatif de la cornée.

10. Système selon l'une quelconque des revendications précédentes, comportant des appareils amont ou aval destinés à effectuer un diagnostic de l'oeil, qui sont réalisés sous la forme de
- lampes à fentes,
- d'ensembles optiques destinés à effectuer une mesure 3D et à assurer un contrôle géométrique de type Scheimpflug, ou sous la forme
- d'ensembles destinés à effectuer une tomographie à cohérence optique (OCT), une topographie, un diagnostic par front d'onde ou une pachymétrie.

11. Système selon l'une quelconque des revendications précédentes, dans lequel,
- à une troisième ou à une quatrième position préférentielle, l'oeil à traiter du patient se trouve dans une zone de mesure ou d'observation d'un appareil amont ou aval destiné à effectuer un diagnostic de l'oeil.

12. Système selon l'une quelconque des revendications précédentes, **caractérisé par** au moins une position d'entrée et de sortie prévue du patient qui est différente des positions préférentielles.

13. Système selon l'une quelconque des revendications précédentes, dans lequel
- les moyens de modification de position de l'installation de mise en place du patient (3) comportent au moins un entraînement de positionnement,
- l'entraînement de positionnement est relié à une unité de commande de positionnement,
- des instructions de réglage sont envoyées par l'unité de commande de positionnement à l'entraînement de positionnement, et
- il est prévu des dispositifs permettant de contrôler et de valider des positions prédéterminées.

14. Système selon la revendication 13, dans lequel l'unité de commande de positionnement est équipée
- d'un module de saisie destiné à saisir manuellement des instructions de réglage, de préférence une manette, ou
- une unité de régulation destinée à générer automatiquement l'instruction de réglage sur la base des grandeurs de mesure de déplacement ou de force déterminées par des capteurs de valeurs de mesure.

15. Système selon la revendication 14, **caractérisé en ce qu'**il est prévu, pour détecter des grandeurs de mesure de déplacement, un système de mesure de position relié à l'unité de commande de positionnement et effectuant une mesure incrémentielle ou absolue ou un système de mesure de position optique comportant un module à caméra.

16. Système selon l'une quelconque des revendications 12 à 15, dans lequel l'unité de commande de positionnement est reliée à un dispositif de positionnement entièrement automatisé du patient ou de l'oeil, qui comporte une unité de commande d'entraînement assistée par navigation équipée d'éléments destinés à la détection de la position de l'oeil et au suivi de la position de l'oeil et de capteurs destinés à détecter des obstacles et d'un module de commande relié auxdits capteurs et aux entraînements de positionnement permettant de contourner des obstacles.

17. Système selon l'une quelconque des revendications 10 à 16, dans lequel il est prévu, pour le positionnement du patient :
- un appuie-tête destiné au patient et un verre de contact pouvant être placé sur l'oeil et maintenu de manière mobile et à travers lequel le faisceau laser de traitement est dirigé vers l'oeil,
- un capteur de valeur de mesure détectant la force avec laquelle le verre de contact repose sur l'oeil, et
- un mécanisme de sécurité qui comporte un capteur de valeur de mesure de force et au moyen duquel l'appuie-tête et le verre de contact sont écartés l'un de l'autre lorsque le verre de contact exerce une force excessive sur l'oeil.

18. Système selon la revendication 17, **caractérisé en ce que** le mécanisme de sécurité ne déclenche l'écartement que lorsque la force dépasse une valeur limite et **en ce que** lorsque la force exercée est inférieure à ladite limite, les positions relatives de l'appuie-tête et du verre de contact sont fixées l'une par rapport à l'autre.

19. Système selon la revendication 1, caractérisé dans lequel le corps de base est équipé d'une base (18) permettant un guidage forcé d'une séquence de mouvements lors de la variation de position de l'installation de mise en place du patient (3) par rapport aux appareils.

20. Système selon la revendication 19, dans lequel les guidages forcés sont mis en oeuvre sous la forme de trajets d'acheminement permettant un guidage forcé, de préférence sous la forme de rails, dans la base (18).

21. Système selon l'une quelconque des revendications précédentes, dans lequel les moyens de communication comprennent des liaisons permettant la transmission sans fil ou de manière filaire de données sous la forme d'un réseau de poste à poste, de préférence sous la forme d'un réseau à base de serveur ou sous la forme d'un réseau en bus, en étoile ou en anneau.

22. Système selon la revendication 21, dans lequel la transmission des données est effectuée de manière filaire et une partie du réseau prévu à cet effet est logée dans le corps de base.

23. Système selon l'une quelconque des revendications précédentes, dans lequel les appareils sont couplés, à des fins d'alimentation en énergie, à une unité d'alimentation centrale.

24. Système selon la revendication 23, dans lequel l'unité d'alimentation centrale est équipée d'un groupe d'alimentation en énergie de secours.
